(19) Europäisches Patentamt
European Patent Office
Office européen des brevets

(11) Veröffentlichungsnummer: **0 035 119**
**B1**

(12) # EUROPÄISCHE PATENTSCHRIFT

(45) Veröffentlichungstag der Patentschrift: **20.07.83**

(21) Anmeldenummer: **81100651.9**

(22) Anmeldetag: **29.01.81**

(51) Int. Cl.³: **C 07 H 17/04**, C 12 P 19/60, C 12 P 17/18, C 07 D 493/10, A 61 K 31/35, A 23 K 1/16 // (C12P19/60, C12R1/365),(C12P17/18, C12R1/365),(C07D493/10, 311/00, 307/00)

(54) **Polyätherverbindungen und ihre Herstellung, entsprechende Präparate und die Verwendung dieser Produkte als Heilmittel oder wachstumsfördernde Mittel bei Wiederkäuern.**

(30) Priorität: **30.01.80 US 116696**

(43) Veröffentlichungstag der Anmeldung: **09.09.81 Patentblatt 81/36**

(45) Bekanntmachung des Hinweises auf die Patenterteilung: **20.07.83 Patentblatt 83/29**

(84) Benannte Vertragsstaaten: **AT BE CH DE FR GB IT LI LU NL SE**

(56) Entgegenhaltungen: **THE JOURNAL OF ANTIBIOTICS, Band 32, Nr. 10, 1979 Seiten 79–96**

(73) Patentinhaber: **F. HOFFMANN-LA ROCHE & CO. Aktiengesellschaft, CH-4002 Basel (CH)**

(72) Erfinder: **Liu, Chao-Min, 36 Rockledge Place, Cedar Grove, N.J. (US)**
Erfinder: **Prosser, Barbara, 146 Darling Avenue, Bloomfield, N.J. (US)**
Erfinder: **Westley, John, 91 South Mountain Avenue, Cedar Grove, N.J. (US)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Polyätherverbindungen und ihre Herstellung, entsprechende Präparate und die Verwendung dieser Produkte als Heilmittel oder wachstumsfördernde Mittel bei Wiederkäuern.

Die vorliegende Erfindung betrifft neue Polyätherverbindungen, d.h. die Antibiotika X-14868A und X-14868B der allgemeinen Formel

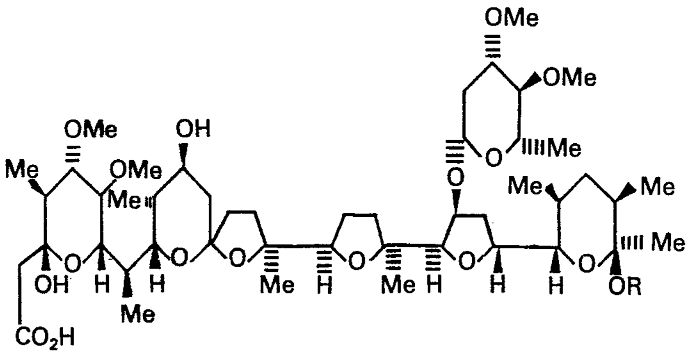

I

in der R Wasserstoff oder Methyl darstellt, und ihre pharmazeutisch verträglichen Salze. Die Erfindung umfasst ebenfalls das neue Antibiotikum X-14868C sowie seine pharmazeutisch verträglichen Salze, wovon das Natriumsalz die folgenden physikalischen Eigenschaften besitzt:

1) Ein Infrarotspektrum gemäss Fig. 3;
2) einen Schmelzpunkt von 172– 175 °C
3) eine spezifische Drehung von

| [α]D Chloroform | Methanol |
|---|---|
| +49,6° | +30,5° und |

4) eine Mikroanalyse von

| %C | %H | %Na |
|---|---|---|
| 57,13 | 8,58 | 2,36 |

sowie das neue Antibiotikum X-14868D und seine pharmazeutisch verträglichen Salze, wovon das Natriumsalz die folgenden physikalischen Eigenschaften besitzt:

1) Ein Infrarotspektrum gemäss Fig. 4;
2) einen Schmelzpunkt von 194–195°
3) eine spezifische Drehung von

| [α]D Chloroform | Methanol |
|---|---|
| +41,1° | +29,2° und |

4) eine Mikroanalyse von

| %C | %H | %Na |
|---|---|---|
| 60,32 | 8,80 | 3,40 |

Die erwähnten vier antibiotischen Verbindungen werden erfindungsgemäss durch ein Verfahren erhalten, welches dadurch gekennzeichnet ist, dass man eine Subkultur des Stammes Nocardia X-14868 ATCC 31585 in einer Kohlenhydrate und stickstoffhaltige Nährstoffe enthaltenden wässrigen Lösung unter submersen aeroben Bedingungen kultiviert und anschliessend die Endprodukte aus der Lösung abtrennt und isoliert.

Das in Formel I verwendete Symbol Me stellt die Methylgruppe dar.

Der die erfindungsgemässen Antibiotika erzeugende Organismus ist eine neue Spezies mit der Bezeichnung Nocardia sp. X-14868. Eine Kultur des lebenden Organismus mit der Laborbezeichnung X-14868 wurde bei American Type Culture Collection, Rockville, Maryland, USA unter der Identifizierungsnummer ATCC 31585 deponiert. Die Kultur wurde als Nocardiastamm identifiziert.

Der neue Mikroorganismus wurde aus Seesand in Colloroy, Australien isoliert. Ein representativer Stamm von Nocardia sp. X-14868 besitzt folgende Merkmale:

Allgemeine Kennzeichen: Nocardia sp. X-14868 ist gekennzeichnet durch fehlende Luftsporenbildung, Fragmentierung des gram-positiven Substratmycels nach mehreren Tagen der Bebrütung sowie durch die Zusammensetzung ihrer Zellwände, welche aus Meso-diaminopimelinsäure, Galaktose und Arabinose bestehen.

Wachstumskennzeichen: Der Mikroorganismus wurde auf Standard Difco ISP-Medien gezüchtet, wie sie in Shirling and Gottlieb: Methods for Characterization of Streptomyces Species, Intern. J. System. Bacteriol., 16, Seiten 313–400, 1966, beschrieben sind sowie auf verschiedenen weiteren Medien gemäss nachstehender Liste:

Hefeextrakt:

Hefeextrakt 1,0%; Glukose 1,0%; Agar 1,5%; pH 6,8

Glukose-Hefeextrakt-Pepton:

Glukose 0,3%; Hefeextrakt 0,5%; Pepton 0,5%; $CaCO_3$, 0,75%; Agar 1,5%; pH 7,0

Glukose-Asparagin:

Glukose 1,0%; Asparagin 0,05%; $K_2HPO_4$ 0,05%; Agar 1,5%; pH 6,8

Saccharose-Nitrat:

Saccharose 1,0%; $NaNO_3$ 0,2%; $K_2HPO_4$ 0,1%; $MgSO_4.7H_2O$ 0,05%; KCl 0,05%; Agar 1,5%.

Für weitere Tests verwendete Medien werden in den nachstehenden Publikationen beschrieben:

| Test | Publikationen |
|---|---|
| A. Kochsalzverträglichkeit<br>B. Caseinhydrolyse | Gordon and Smith, «Rapidly Growing Acid Fast Bacteria», J. Bacteriol., 66:41–48, 1953 |
| C. Nitratreduktion<br>D. Gelatine (modifiziert mit Actinomycesmedium (Difco) plus 2,0% Agar in Fleischinfusionsagar)<br>E. Stärke (Actinomycesmedium (Difco) plus 0,25% lösliche Stärke und 2,0% Agar).<br>F. Wirkung auf Lackmusmilch (Difco) | Skerman, «A Guide to the Identification of the Genera of Bacteria», Williams and Williams Co., Baltimore, 1967. |
| G. Lysozymresistenz<br>H. Penicillinempfindlichkeit (Platten zu 10 Einheiten) | Gordon, «Some Criteria for the Recognition of Nocardia», J. Gen. Microbiol., 45:355–364, 1966. |

Die Versuche werden bei 28 °C und 37 °C für fast sämtliche Medien durchgeführt. Die Farbe nach zwei- und vier-wöchiger Bebrütung bestimmt.

Die Kohlenstoffverwertung wurde nach der Methode von Shirling and Gottlieb (oben) mit Hilfe von ISP-9 (Difco) Medium bestimmt.

Eine 48 Stunden alte ISP-1 Brühekultur von X-14868 wurde zentrifugiert und homogenisiert zwecks Erhalts einer gewaschenen Suspension für Beimpfung.

Die Fähigkeit des Organismus, bei 10, 28, 36, 45 und 50 °C zu wachsen, wurde durch Beimpfung von ISP-1 (Difco) Medium untersucht. Die Zellwandanalyse wurde nach der Methode von Bekker et al., durchgeführt (Appl. Microbiol., 12, 421–423, 1964). Für die Bestimmung des Zuckergehalts der Zellwand wurde die Methode von Lechevalier und Lechevalier, Actinomycetales, Ed.H. Prauser, Gustav Fischer, Jena, Seiten 311–316, 1970, verwendet. Nocardomycolsäureanalyse wurde mittels einer leichten Modifikation der Methode von Lechevalier, Lechevalier und Horan, Can. J. Microbiol. 19:965–972, 1973, durchgeführt.

Ergebnisse

Mikroskopische Untersuchung: Der Stamm X-14868 erzeugt ein Substratmycel, welches nach mehreren Tagen fragmentiert, wodurch ausgiebige Mycelbildung erfolgt. Der Organismus erzeugt ein Luftmycel bestehend aus seilähnlichen Büscheln; jedoch wurden keine Sporen gefunden.

Zellwandanalyse: Die Zellwand enthält das Mesoisomere von Diaminopimelinsäure wie auch Galaktose und Arabinose (Zellwand Typ IV der Klassifikation von Lechevalier et al., Adv. Appl. Microbiol., 14, 47–72, 1971). Der Organismus scheint Nocardomycolsäure zu erzeugen. Diese morphologischen und chemischen Kriterien ordnen den Mikroorganismus X-14868 in die Gattung Nocardia ein.

Makroskopische Untersuchungen: Das Wachstumsverhalten, der Sporulationsgrad, die Farbe des Luftmycels sowie die Farbe der Rückseite des Mycels und die Anwesenheit von durch X-14868 auf verschiedenen festen Nährmedien nach vierwöchiger Brütung bei 28 °C erzeugtem löslichem Pigment werden in der nachstehenden Tabelle 1 zusammengefasst.

Tabelle 1. Kulturmerkmale von Nocardia X-14868

| Agarmedium | Wachstum und Luftmycel | Farbe des Luftmycels[a] | Farbe der Rückseite des Mycels[a] |
|---|---|---|---|
| Hefe-Malzextrakt (ISP-2) | mittleres bis reichliches Wachstum; etwas Luftmycel an isolierten Rändern, lederartiges Wachstum | b (austernweiss) | 2 ie (hell senfbraun) |
| Hafermehl (ISP-3) | mittleres Wachstum; mittleres Luftmycel | b (austernweiss) | 3 dc (naturfarben) |
| Anorganische Salze/Stärke (ISP-4) | spärliches Wachstum; spärliches Luftmycel | b (austernweiss) | 2 dc (naturfarben) |

(Fortsetzung)

Tabelle 1. Kulturmerkmale von Nocardia X-14868

| Agarmedium | Wachstum und Luftmycel | Farbe des Luftmycels[a] | Farbe der Rückseite des Mycels[a] |
|---|---|---|---|
| Glycerol-Asparagin (ISP-5) | schwaches Wachstum; fast kein Luftmycel; Substratmycel | b (austernweiss) | 2 ge (bräunlich) |
| Hefeextrakt | reichliches Wachstum; spärliches Luftmycel; lederartiges Wachstum | b (austernweiss) | 3 ge (beigefarben) |
| Glukose-Hefeextrakt-Peptone | mittleres Wachstum; spärliches Luftmycel am Rande; lederartiges Wachstum; braunes lösliches Pigment | b (austernweiss) am Rande | 3 ge (beigefarben) |
| Glukose-Asparagin | mässiges Wachstum; etwas Luftmycel; lederartiges Wachstum | 2dc (naturfarben) | 3 ge (beigefarben) |
| Saccharose-Nitrat | spärliches Wachstum; mittleres Luftmycel | b (austernweiss) | durchsichtig c (hellgrau) |

NB. Keine Sporen wurden gefunden im Luftmycel irgendeines der Medien nach 4 Wochen Bebrütung.
[a] Die Farbskala wurde Color Harmony Manual, 4th ed. 1958 (Container Corporation of America, Chicago) entnommen.

Physiologische Kennzeichen: Der Mikroorganismus X-14868 hydrolysiert Gelatine und Casein, jedoch nicht Stärke. Die Kultur ist Penicillin-resistent (Platte zu 10 Einheiten) sowohl gegen 0,005% Lysozymbrühe nach der Testmethodik von Gordon, J. Gen. Microbiol., 45, 355–364, 1966. Der Mikroorganismus peptonisiert Lackmusmilch vollständig. Keine Melaninproduktion entsteht auf ISP 1, 6 oder 7.

Das Ergebnis der Kohlenstoffverwertung auf ISP-9 (Difco) durch den Mikroorganismus X-14868 bei 28 °C nach einmonatiger Bebrütung ist in Tabelle 2 zusammengestellt.

Tabelle 2. Kohlenstoffverwertung durch X-14868

| Kohlenstoffquelle | Kennzeichen[a] |
|---|---|
| Kontrollprobe (kein Kohlenstoff) | – |
| D-Glukose | ++ |
| D-Xylose | – |
| L-Arabinose | – |
| L-Rhamnose | – |
| D-Fructose | ± |
| D-Galaktose | + |
| Raffinose | – |
| D-Mannit | – |
| i-Inosit | – |
| Salicin | ± |
| Saccharose | – |
| Cellulose | – |

[a]–, negative Reaktion; ± zweifelhafte Reaktion; + kräftigeres Wachstum als auf Kontrollprobe aber weniger als auf Glukose; ++ positive Reaktion, gleichwertig mit dem Wachstum auf Glukose.

In Tabelle 3 sind einige diagnostisch wichtige, meistens metabolische Eigenschaften zusammengestellt.

Tabelle 3. Eigenschaften von X-14868

| Test | Ergebnis |
|---|---|
| ISP 1, Verdunkelung | – |
| ISP 6, Verdunkelung | – |
| ISP 7, Melanin | – |
| Hydrolyse von Casein | ++ |
| Hydrolyse von Gelatine | ++ |
| Hydrolyse von Stärke | – |
| NaCl-Toleranz% | 5% |
| Temperatur, bei der Wachstum beobachtet wurde | 28 und 36 °C |
| Pigment der Rückseite | keines |
| Lösliches Pigment | braun auf Glukose/Hefeextrakt/Pepton |
| Penicillinempfindlichkeit (Platte zu 10 Einheiten) | – |
| Nitratreduktion | ++ |
| Gramfärbung | + |
| Säurefestigkeit | – |
| Catalase | + |
| Diaminopimelinsäure | meso-Isomeres |
| Zellwandzucker | Galaktose, Arabinose, Ribose |
| Erzeugung von Nocardomycolsäure | + |

Der Mikroorganismus X-14868 kann aufgrund

der biochemischen Kennzeichen in der nachstehenden Tabelle 4 von anderen Nocardiaspezies unterschieden werden. Diese Spezies wurde zum Vergleich ausgewählt, weil sie die einzige Gruppe innerhalb der Gattung Nocardia darstellt, welche X-14868 einigermassen phänotypisch ähnelt.

Zwar sind in Tabelle 4 wenig biochemische Daten vorhanden für vier der Spezis, d.h. N. transvalensis, N. formicae, N. petroleophila und N. saturnea. Diese Spezies sind jedoch von X-14868 sehr verschieden. N. transvalensis gilt als der Spezies N. brasiliensis, (in Tabelle 4 aufgeführt) sehr ähnlich. N. formicae ist gegebenenfalls fälschlicherweise in der Gattung Nocardia plaziert und ähnelt mehr der Gattung Streptomyces. N. petroleophila und N. saturnea sind zwei sehr ausgeprägte Spezies mit geringer Ähnlichkeit zu anderen Nocardiaspezies. Arten aus diesen beiden Spezies können in kohlenstofffreiem Medium unter Verwendung von atmosphärischem Kohlenstoffdioxid leben; dies ist bei den anderen Spezies, einschliesslich X-14868, nicht der Fall.

Tabelle 4.
Vergleich von X-14868 mit anderen Nocardiaspezies

| | N.farcinica | N.otitidis-caviarum | N.brasiliensis | N.asteroides | N.transvalensis | N.formicae | N.coeliaca | N.polychromogenes | N.paraffinae | N.petroleophila | N.saturnea | N.kuroishii | X-14868 |
|---|---|---|---|---|---|---|---|---|---|---|---|---|---|
| Kohlenstoffverwertung | + | + | + | + | | | + | + | + | | | + | + |
| L-Arabinose | | | | | | | | | | | | | – |
| L-Rhamnose | | | | | | | | | | | | | – |
| D-Fructose | + | + | + | + | | | | + | + | | | + | ± |
| Galactose | | | + | | | | | | | | | | |
| Mannit | | + | + | + | | | | + | | | | + | – |
| Inosit | | | + | | | | | | | | | | – |
| Saccharose | | | | | | | | | | | | + | – |
| Maltose | + | + | + | + | | | + | + | | | | | – |
| Mannose | + | | + | + | | | + | + | + | | | + | ± |
| Glycerin | | + | | + | | | + | + | + | | | + | – |
| Laktose | | | | | | | | | | | | + | |
| Paraffin | + | + | + | + | | – | + | + | + | | | | + |
| Acetat | + | + | + | + | | | + | + | + | | | | – |
| Butyrat | + | + | + | + | | | + | + | + | | | | – |
| Malat | + | | + | + | | | + | + | | | | | + |

(Fortsetzung)

Tabelle 4.
Vergleich von X-14868 mit anderen Nocardiaspezies

| | N.farcinica | N.otitidis-caviarum | N.brasiliensis | N.asteroides | N.transvalensis | N.formicae | N.coeliaca | N.polychromogenes | N.paraffinae | N.petroleophila | N.saturnea | N.kuroishii | X-14868 |
|---|---|---|---|---|---|---|---|---|---|---|---|---|---|
| Propionat | + | + | + | + | | | + | | + | | | | – |
| Pyruvat | + | + | + | + | | | + | + | + | | | | + |
| Succinat | + | | + | + | | | | + | + | | | | + |
| Citrat | | | + | | | | | | | | | | ± |
| Nitratreduktion | | + | + | + | – | + | – | + | + | – | – | – | + |
| Xanthinhydrolyse | | + | – | – | | | + | | | | | | + |
| Gelatinehydrolyse | | | + | – | | + | + | – | – | – | – | – | + |

Die pharmazeutisch verträglichen Salze des Antibiotikums X-14868A können nach an sich bekannten Methoden hergestellt werden, beispielsweise durch Waschen der freien Säure in Lösung mit einer geeigneten Base oder einem geeigneten Salz. Beispiele für solche pharmazeutisch verträgliche basische Substanzen sind Alkalimetallbasen, wie Natriumhydroxid, Kaliumhydroxid, Lithiumhydroxid und dergleichen; Erdalkalimetallbasen wie Calciumhydroxid, Bariumhydroxid und dergleichen; und Ammoniumhydroxid. Alkalimetall- oder Erdalkalimetallsalze, welche verwendet werden können, sind beispielsweise Carbonate, Bicarbonate und Sulfate.

Nocardia X-14868 erzeugt nach Züchtung unter geeigneten Bedingungen die Verbindung X-14868A. Die dafür benötigte Gärlösung wird durch Beimpfung des Nährmediums mit Sporen oder Mycel des Mikroorganismus hergestellt; die Nährlösung wird anschliessend unter aeroben Bedingungen kultiviert. Es ist ebenfalls möglich, die Kultivierung auf einem festen Nährboden durchzuführen, jedoch wird für die Erzeugung grösserer Mengen ein flüssiges Medium bevorzugt. Die Gärtemperatur kann im weiten Bereich, 20–35 °C, variiert werden, jedoch sind Temperaturen von 26–30 °C und im wesentlichen neutrales pH bevorzugt. Für die submerse, aerobe Fermentation kann das Nährmedium als Kohlenstoffquelle ein im Handel erhältliches Glyceridöl oder ein Kohlenhydrat, wie z.B. Glycerin, Glukose, Maltose, Lactose, Dextrin, Stärke etc. in reinem oder rohem Zustand enthalten; als Stickstoffquelle kommen organische Materialien, wie z.B. Soyabohnenmehl, lösliche Rückstände aus der Brennerei, Erdnussöl, Baumwollsamenmehl, Fleischextrakt, Pepton, Fischmehl, Hefeextrakt, Maisquellwasser etc. in Betracht. Erwünschtenfalls können auch anorganische Stickstoffquellen verwendet werden, z.B. Nitrate und Ammoniumsalze. Es können auch Mineralsalze verwendet werden, wie z.B. Ammoniumsulfat, Magnesiumsulfat, Natriumchlorid, Kaliumchlorid, Kaliumphosphat, Calciumcarbonat und dergleichen, sowie Puffer wie Natriumcitrat, Phosphate und Spurenmengen von Schwermetallsalzen. Wenn die Fermentation unter belüfteten submersen Bedingungen durchgeführt wird, verwendet man vorzugsweise ein Schaumbekämpfungsmittel, wie flüssiges Paraffin, fettes Öl oder Siliconprodukte. Mehr als eine Art von Kohlenstoffquelle, Stickstoffquelle bzw. Schaumbekämpfungsmittel können verwendet werden.

In der nachstehenden Tabelle 5 wird die antimikrobielle Wirksamkeit des Antibiotikums X-14868A sowie der drei in geringeren Mengen erhältlichen Komponenten X-14868B, X-14868C und X-14868D angegeben.

Tabelle 5

| Mikroorganismus | Mindesthemmkonzentration (mcg/ml) X-14868A | X-14868B | X-14868C | X-14868D |
|---|---|---|---|---|
| G (+)-Cocci | | | | |
| Streptococcus faecium ATCC 8043 | 0.313 | 1.57 | 0.79 | 0.19 |
| Staphylococcus aureus ATCC 6538p | 6.25 | 6.25 | 62.5 | 12.5 |
| Sarcina lutea ATCC 9341 | 12.5 | 12.5 | 250 | 62.5 |
| G (+)-Stäbchen-Bacillus | | | | |
| Bacillus megaterium ATCC 8011 | 12.5 | 7.5 | 125 | 61.5 |
| Bacillus sp. E ATCC 27859 | 0.39 | 0.79 | 6.25 | 1.57 |
| Bacillus subtilis NRRL 558 | 12.5 | 25 | 250 | 62.5 |
| Bacillus sp. ATCC 27860 | 6.25 | 12.5 | 125 | 25 |
| G (+)-Filamente | | | | |
| Mycobacterium phlei ATCC 355 | 12.5 | 25 | 250 | 62.5 |
| Streptomyces ceilulosae ATCC 3313 | 25 | 25 | 500 | 125 |
| Pilze | | | | |
| Paecilomyces varioti ATCC 26820 | 250 | 500 | * | * |
| Penicillum digitatum ATCC 26821 | 1000 | 1000 | * | * |
| Hefen | | | | |
| Candida albicans NRRL 477 | 250 | 100 | * | * |
| Saccnaromyces cerevisae NRRL 4226 | 1000 | * | * | * |

* Umwirksam bei 1 mg/ml

Aus Tabelle 5 geht hervor, dass das Antibiotikum X-14868A sowie die drei in geringeren Mengen erhältlichen Komponenten das Wachstum gewisser grampositiver Bakterien beeinträchtigen. Sie sind deshalb verwendbar in Waschlösungen für sanitäre Zwecke, wie z.B. zum Händewaschen und Reinigung von Ausrüstungen, Böden bzw. Einrichtungen von kontaminierten Räumen oder Laboratorien.

In der nachstehenden Tabelle 6 wird die Anticoccidiosewirkung des erfindungsgemässen Antibiotikums X-14868A im Vergleich zu uninfizierten, unbehandelten Kontrolle sowie im Vergleich zu einer infizierten unbehandelten Kontrolle aufgeführt.

Tabelle 6

| Wirkstoff | Konz.% | Gewichtszunahme% | Mortalität% | Durchschnittlicher Infektionsgrad Intestinalverletzungen Obere | Mittlere | Caecale | Durchschnitt |
|---|---|---|---|---|---|---|---|
| Uninfizierte unb. Kontrolle | | 100 | 0 | 0.0 | 0.0 | 0.0 | 0.0 |
| Infizierte unb. Kontrolle | | 37 | 20 | 2.7 | 2.0 | 3.0 | 2.6 |
| X-14868A, Natriumsalz Lot-1 | 0.002 | 39 | 0 | 0.0 | 0.0 | 0.0 | 0.0 |
| | 0.001 | 81 | 0 | 0.2 | 0.0 | 0.0 | 0,07 |
| | 0.0005 | 96 | 0 | 0.4 | 0.3 | 0.0 | 0.2 |
| X-14868A, Natriumsalz Lot-1A | 0.002 | 60 | 0 | 0.0 | 0.0 | 0.0 | 0.0 |

Versuchsmethodik: Es werden 10 Küken pro Dosis eingesetzt. 10 Küken werden als Gewichtskontrolle und 10 Küken als infizierte Kontrolle verwendet. Die Prüfsubstanz wird 48 Stunden vor der Infektion gegeben. 1 g der Prüfsubstanz wird in einem mechanischen Mischer mit dem benötigten Kükenfutter vermischt, um die gewünschte Dosierung zu erreichen. Die Infektion besteht aus etwa 300 000 Oocysten, peroral mit Hilfe einer Pipette verabreicht. Der Versuch dauert 6 Tage, wonach die überlebende Tiere in der Autopsie auf grobe caecale Verletzungen untersucht werden. Die Zahl der überlebenden Tiere und die Zahl der caecalen Verletzungen werden ermittelt. Die Ergebnisse werden als «durchschnittlicher Infektionsgrad» ausgedrückt. Bei einem durchschnittlichen Infektionsgrad von weniger als 2,5 wird die zugehörige Dosis als signifikant wirksam angesehen.

Die erfindungsgemässen coccidiostatischen Mittel, welche als Wirkstoff das Antibiotikum X-14868A oder eines seiner pharmazeutisch verträglichen Salze oder auch die getrocknete unfiltrierte Gärbrühe enthalten, werden durch Vermischen des Wirkstoffes mit einem inerten Träger hergestellt. Der inerte Träger kann aus Futter, Ersatzmaterialien etc. bestehen. Mit dem Ausdruck «inerter Träger» ist ein Material gemeint, das als antiparasitäres Mittel, z.B. als Coccidiostaticum, nicht wirkt, in Bezug auf den Wirkstoff sich inaktiv verhält und des weiteren für die behandelten Tiere unschädlich ist.

Das erfindungsgemässe Mittel unterdrückt die Coccidiose bei Nutzgeflügel, insbesondere Puten oder Küken, das für diese Krankheit besonders anfällig ist. Nach der oralen Verabreichung des Mittels in der Form eines Futterzusatzes wird die Coccidiose entweder verhindert oder, bei bereits vorhandener Krankheit, geheilt. Ein weiterer Vorteil besteht darin, dass die behandelten Tiere, verglichen mit Kontrolltieren, entweder ihr Gewicht behalten oder sogar ihr Gewicht erhöhen. Die erfindungsgemässen Mittel kontrollieren somit nicht nur die Coccidiose, sondern sie wirken darüber hinaus als wachstumsfördernde Mittel.

Die Konzentration des Wirkstoffes in Futtermitteln kann den individuellen Bedürfnissen entsprechend variiert werden. Beispielsweise kann ein Futterprämix oder ein vollständiges Futter mit genügend Wirkstoff angereichert werden, um etwa 0,0003 bis etwa 0,001 Gewichtsprozent des täglichen Futterkonsums zu enthalten. Bevorzugt verwendet man etwa 0,0005 bis 0,001 Gewichtsprozent. Im allgemeinen genügen 0,001 Gewichtsprozent des Wirkstoffes, um die Coccidiose erforderlich zu unterdrücken bzw. zu bekämpfen. Grössere Mengen als 0,001% sind zwar wirksam, sie zeigen jedoch im allgemeinen keine Vorteile gegenüber 0,001% und können in einigen Fällen das Wachstum, die Futterverwertung und die Mortalität beeinträchtigen.

Die Konzentration von 0,001 Gewichtsprozent ist als obere Grenze deshalb bevorzugt, weil unterhalb dieser Grenze die Kosten bezogen auf die Wirkung am niedrigsten sind. Konzentrationen unter 0,0003 Gewichtsprozent sind für die Bekämpfung der Coccidiose nicht genügend. Eine bevorzugte untere Grenze liegt bei 0,0005 Gewichtsprozent weil diese untere Grenze optimale Wirkung gewährleistet. Die am meisten bevorzugte Konzentration, d.h. etwa 0,0005 Gewichtsprozent des täglichen Futterkonsums ist besonders wirksam, weil sie einen maximalen Effekt bei minimaler Dosis erreicht.

Für die Behandlung oder Verhinderung von Coccidiose kann der Wirkstoff zunächt in die Form eines Konzentrats, eines Futterzusatzprämixes oder eines Futterzusatzes gebracht werden. Aus diesen können durch Verdünnen Vollfutter oder Zusatzfutter hergestellt werden. Beispiele von Trägern sind: handelsübliche Geflügelfutter, gemahlene Cerealien, Nebenprodukte der Müllereiindustrie, Pflanzenproteinkonzentrate (Soya, Erdnuss etc.), Nebenprodukte von Fermentationen, Salz, Kalkstein, anorganische Verbindungen usw. oder Mischungen hiervon. Flüssige Dispersionen können unter Verwendung von Wasser oder pflanzlichen Ölen, bevorzugt unter Einbeziehung eines oberflächenaktiven Mittels oder eines Emulgators usw. wie Äthylendiamintetraessigsäure etc. und von Lösungsvermittlern hergestellt werden.

Auch andere Träger oder Ersatzmaterialien können als inerte Träger verwendet werden, sofern sie sich gegenüber dem Wirkstoff inert verhalten und gegenüber dem zu verabreichenden Tier untoxisch sind.

Typische Geflügelfutter, die mit dem Wirkstoff vermengt werden können, enthalten z.B. Getreideprodukte wie Mais, Weizen, Hafer, Gerste, Milo, Hafermehl, Nachmehl (z.B. Weizennachmehl) usw; stabilisierte Fette; Pflanzenproteine, wie Soyamehl, Maisglutenmehl, Erdnussmehl usw; Tierproteine wie Fischmehl, wasserlösliche Anteile von Fischen («fish solubles») Fleischabfälle usw; Quellen für UGF («unidentified growth factor») und andere B-Vitaminträger wie Trockenmilchprodukte, getrocknete Brauereihefe, getrocknete lösliche Rückstände der Destillationsindustrie, lösliche Rückstände von Fermentationen etc; Lucernegrünmehl; und verschiedene Spezialzusatzmittel, wie Riboflavin, Vitamin $B_{12}$, Calciumpantothenat, Niacin, Cholin, Vitamin K, Vitamin E, stabilisiertes Vitamin A, Vitamin $D_3$ (aktivierte tierische Sterole), Calcium- und Phosphorsupplemente wie z.B. Dicalciumphosphat, dampfbehandeltes Knochenmehl, defluoriertes Phosphat, Kalkstein etc., jodiertes Salz, Mangansulfat, Zinkcarbonat, Methionin oder dessen Hydroxyanalogon, Antioxidantien sowie auch weitere, antibiotisch wirksame Futterzusätze.

Die in geringeren Mengen erhältlichen Komponenten X-14868B, C und D besitzen ebenfalls Anticoccidiose-Wirkung in vitro. X-14868B zeigt diese Wirksamkeit auch in vivo.

Das Antibiotikum X-14868A ist wirksam als wachstumsförderndes Mittel bei Wiederkäuern, d.h. Tieren mit einer Pansenfunktion, z.B. Rindvieh. Eine Erläuterung des Mechanismus, wodurch das Futter in wiederkäuenden Tieren verdaut, abgebaut und metabolisiert wird, wird in der U.S.-Patentschrift Nr. 3 839 557 gegeben; es wird

dort die Verwendung gewisser Antibiotika zur Verbesserung des Futternutzeffektes bei Wiederkäuern beschrieben. Wirtschaftlich wichtige Wiederkäuer sind Rind, Schaf und Ziege.

Die Wirksamkeit des Antibiotikums X-14868A zur Modifizierung des Verhältnisses der im Pansen erzeugten flüchtigen Fettsäuren (wodurch die Futterverwertung verbessert wird) ist aus den nachstehenden in vitro Versuchsergebnissen ersichtlich.

Es wird Pansenflüssigkeit eines Stieres mit einer Pansen-Fistel gewonnen. Der Stier erhält folgendes Futter:

| | |
|---|---|
| Mais: | 89,93% |
| Alfalfamehl: | 5,000% |
| Soyabohnenölmehl: | 3,00% |
| Kalk: | 0,80% |
| NaCl: | 0,60% |
| Dicalciumphosphat: | 0,50% |
| Spurenmineralien: | 0,025% |
| Vitamin prämixzusätze: | 0,1% |
| Vitamin A, TIU: | 4,0003 |
| Vitamin $D_3$, IU: | 0,801 |
| Vitamin E, TIU: | 3,002 |

Die Pansenflüssigkeit wird unmittelbar durch ein Sieb gesiebt. Die Flüssigkeit wird einer Fermentation unterworfen wie folgt:

75 ml der erhaltenen Flüssigkeit in einen 250 ml Kolben vorgelegt, der ausserdem folgende Zusätze enthält:

1 g 80%:20% fein gemahlenes Korn: Heu;

1 ml einer 18%igen wässrigen Glukoselösung (1 mMol pro Kolben);

1,5 ml einer 3,1%igen wässrigen Harnstofflösung (0,76 mMol pro Kolben);

je 60 Micromol der 10 essentiellen Aminosäuren (Arginin, Histidin, Leucin, Methionin, Threonin, Valin, Lysin, Isoleucin, Phenylalanin, Tryptophan);

1 ml einer wässrigen Lösung der Testsubstanz um entweder 10 oder 25 mg/ml zu erhalten (berechnetes totales Volumen der Fermentationsmischung: 80 ml).

Jeder Kolben wird bei 38 °C in einem mit Abzug versehenen, schüttelnden Wasserbad bebrütet. Kohlendioxid wird kontinuierlich durch den Abzug geleitet. Nach 4-stündiger Bebrütung werden 10 ml der Fermentationsflüssigkeit 20 Minuten bei 14 000 upm (etwa 30 000 g) zentrifugiert. 1 ml einer 25%igen Metaphosphorsäurelösung, enthaltend 23 Micromol 2-Methylvaleriansäure, wird mit 3 ml der aus der Zentrifugierung erhaltenen überstehenden Phase versetzt. Die erhaltene Flüssigkeit wird bei Zimmertemperatur 30 Minuten stehen gelassen. Die Flüssigkeit wird durch ein 0,22 Millimicron Filter filtriert und bis zur gaschromatographischen Analyse flüchtiger Fettsäuren in der Kälte aufbewahrt.

Gaschromatographische Analyse von vier in vitro Kontrollgärungen und 2 Gärungen mit je 10 bzw. 25 Microgramm Antibiotikum X-14868A pro ml sind aus der folgenden Tabelle ersichtlich:

Tabelle 7

Molverhältnis von Propionat ($C_3$) zu Acetat ($C_2$) plus n-Butyrat ($nC_4$)

| Verbindung | Konzentration | Molverhältnis flüchtiger Fettsäuren $C_3/(C_2+nC_4)$ | % der positiven Kontrolle |
|---|---|---|---|
| Negative Kontrolle | 0 | 0.203 | 51.8 |
| X-14868A | 5 ppm | 0.323 | 82.5 |
| | 50 ppm | 0.358 | 91.2 |
| Positive Kontrolle Monensin | 50 ppm | 0.392 | 100.0 |

Die obige Tabelle 7 zeigt, dass das Verhältnis von Propionat zur Summe von Acetat und n-Butyrat wesentlich verbessert wird. Bei vermehrter Bidlung von Propionat eher als Acetat aus den Kohlenhydraten wird die Wirkung der Kohlenhydrate und daher die Futterverwertung erhöht.

Durch Verabreichung des Antibiotikums X-14868A (nachstehend «das Antibiotikum» genannt) wird Ketose verhindert bzw. geheilt; gleichzeitig wird die Futterverwertung verbessert. Die Ursache der Ketose liegt in einer ungenügenden Bildung von Propionat. Eine gegenwärtig empfohlene Behandlungsmethode besteht in der Verabreichung von Propionsäure bzw. von Futtern, welche bevorzugt Propionat erzeugen. Es ist offensichtlich, dass die vermehrte Bildung von Propionat bei der Einnahme von üblichen Futtermitteln die Ketosefälle vermindern wird.

Die einfachste Methode zur Verabreichung des Antibiotikums besteht darin, dass man es mit dem Tierfutter vermischt. Das Antibiotikum kann jedoch auch in anderer Weise verabreicht werden. Es kann beispielsweise zu Tabletten, Tränken, Boli oder Kapseln verarbeitet werden, welche dem Tier in dosierter Form verabreicht werden können. Formulierungen des Antibiotikums in derartigen Dosierungsformen werden nach an sich in der Veterinärpharmazie bekannten Methoden hergestellt.

Kapseln werden in üblicher Weise hergestellt durch Auffüllen von Gelatinekapseln beliebiger Form mit dem Antibiotikum. Erwünschtenfalls kann das Antibiotikum mit einem inerten, festen

Verdünnungsmittel verdünnt werden, wie beispielsweise mit einem Zucker, Stärke oder gereinigter kristalliner Cellulose, um das Volumen aus Zweckmässigkeitsgründen zu erhöhen.

Tabletten werden durch herkömmliche pharmazeutische Methoden hergestellt. Eine Tablette enthält im allgemeinen ausser dem Wirkstoff einen Grundstoff, einen Desintegrator, ein Adsorbens, ein Bindemittel und ein Gleitmittel. Typische Grundstoffe sind z.B. Milchzucker, feiner Puderzucker, Natiumchlorid, Stärke und Mannit. Stärke ist ebenfalls ein guter Desintegrator, wie ebenso Alginsäure. Oberflächenaktive Mittel, wie Natriumlaurylsulfat und Dioctylnatriumsulfosuccinat werden gelegentlich ebenfalls verwendet. Üblicherweise verwendete Adsorbentien sind z.B. Stärke und Milchzucker, während Magnesiumcarbonat bei öligen Substanzen Verwendung findet. Oft verwendete Bindemittel sind Gelatine, Gummi arabicum, Stärke, Dextrin und verschiedene Cellulosederivate. Häufig verwendete Gleitmittel sind z.B. Magnesiumstearat, Talk, Paraffinwachs, verschiedene Metallseifen und Polyäthylenglykol.

Das Antibiotikum kann ebenfalls in Form eines Bolus mit verzögerter Wirkung verabreicht werden. Derartige Boli werden als Tabletten hergestellt, wobei jedoch ein Mittel zur Verzögerung der Auflösung des Antibiotikums zugefügt wird. Boli sind für Freisetzung während längerer Perioden vorgesehen. Die langsame Auflösung wird durch Wahl einer stark wasserunlöslichen Form des Antibiotikums gewährleistet. Beispielsweise können Eisenfeilspäne zugegeben werden, um die Dichte des Bolus zu erhöhen, damit dieser am Boden des Pansens bleibt.

Die Auflösung des Antibiotikums wird verzögert durch Verwendung einer Matrize unlöslichen Materials, in welcher der Wirkstoff eingebettet wird. Beispielsweise können pflanzliche Wachse, gereinigte Mineralwachse und wasserunlösliche, polymere Materialien verwendet werden.

Tränken des Antibiotikums werden zweckmässig durch Wahl einer wasserlöslichen Form des Antibiotikums hergestellt. Wird eine unlösliche Form gewünscht, kann eine Suspension hergestellt werden. Wahlweise kann ein Trank als eine Lösung in einem physiologisch einwandfreien Lösungsmittel, wie beispielsweise Polyäthylenglykol, zubereitet werden.

Suspensionen von unlöslichen Formen des Antibiotikums können in Nicht-Lösungsmitteln hergestellt werden, wie beispielsweise in pflanzlichen Ölen, z.B. Erdnuss-, Mais- oder Sesamöl, in einem Glykol, wie z.B. Propylenglykol oder Polyäthylenglykol, oder in Wasser, je nach der gewählten Form des Antibiotikums.

Geeignete physiologisch verträgliche Zusätze sind notwendig, um das Antibiotikum in Suspension zu halten. Die Zusätze können Verdickungsmittel sein, wie z.B. Carboxymethylcellulose, Polyvinylpyrrolidon, Gelatine oder Alginate. Verschiedene Klassen von Schaumerzeugern dienen dazu, das Antibiotikum in Suspension zu erhalten. Beispielsweise sind Lecithin, Alkylphenol/Polyäthylenoxidaddukte, Naphthalinsulfonate, Alkylbenzolsulfonate und Polyoxyäthylensorbitanester für die Herstellung von Suspensionen in flüssigen Nichtlösungsmitteln verwendbar.

Für die Herstellung von Suspensionen können zusätzlich verschiedene Substanzen, welche die Hydrophilität, Dichte und Oberflächenspannung beeinflussen, verwendet werden. Beispiele sind Silikone, Glykole, Sorbit und Zucker, die als Suspendierungsmittel Verwendung finden.

Das Antibiotikum kann dem Verbraucher als eine Suspension oder als trockenes Gemisch mit entsprechenden Hilfsmitteln zur Verdünnung vor der Verwenung vorgelegt werden.

Das Antibiotikum kann ebenfalls dem Trinkwasser des Wiederkäuers zugeführt werden. Dabei wird eine wasserlösliche oder wassersuspendierbare Form des Antibiotikums dem Trinkwasser in entsprechender Menge zugegeben. Die Zubereitung des Antibiotikums für das Vermischen mit dem Trinkwasser folgt den gleichen Prinzipien wie die Formulierung von Tränken.

Die zweckmässigste Art, das Tier mit dem Antibiotikum zu behandeln, basiert auf der Verabreichung mit dem Tierfutter. Beliebige Tierfutter können verwendet werden, z.B. übliche Trockenfutter, flüssige Futter und tablettierte Futter.

Zur Herstellung eines medikierten Tierfutters wird üblicherweise zuvor eine konzentrierte Vormischung mit dem Antibiotikum hergestellt. Beispielsweise kann eine Vormischung von etwa 2 bis etwa 900 g Antibiotikum pro Kg Vormischung enthalten. Dieser weite Bereich ist durch den benötigten, weiten Konzentrationsbereich für das fertige Futter bedingt. Vormischungen können sowohl in flüssiger als auch in fester Form vorliegen.

Wie oben gezeigt, bewirkt die orale Verabreichung des Antibiotikums eine vorteilhafte Änderung der Erzeugung von Propionat im Verhältnis zu Acetat im Pansen. Es kann deshalb postuliert werden, dass die gleiche Behandlung ebenfalls monogastrische Tiere, die faseriges Pflanzenmaterial im Blinddarm vergären, günstig beeinflusst, denn es kann erwartet werden, dass eine vorteilhafte Veränderung im Propionat – Acetatverhältnis nach oraler Gabe des Antibiotikums auftreten wird. Pferde, Schweine und Kaninchen sind Beispiele von Tieren, welche einen Teil ihres Futters durch Fermentation im Blinddarm verdauen.

Das Antibiotikum X-14868A ist ebenfalls wirksam als Mittel bei der Behandlung bzw. Prophylaxe von Schweinedysenterie. Die Wirkung der Verbindung gegen Treponema hyodysenteriae, den Erreger von Schweinedysenterie, wurde untersucht. Das Ergebnis ist in der nachstehenden Tabelle 8 zusammengestellt.

Tabelle 8

| | | Mindesthemmkonzentration (mcg/ml) |
|---|---|---|
| T. hyodysenterie-Stamm | X-14868A | Ipronidazol |
| 1 | 0,05 | 0,63 |
| 2 | 0,05 | 0,63 |
| 3 | 0,05 | 0,63 |
| 4 | 0,05 | 2,5 |
| Durchschnitt | 0,05 | 1,1 |

Die in geringerer Menge erhältlicher Komponenten X-14868B, C und D besitzen in vitro Anticoccidiosewirkung gegen E. Tenella wie folgt:

| Verbindung | Wirksam gegen E. tenella (µg/ml) |
|---|---|
| X-14868B | 0,1 |
| X-14868C | 10,0 |
| X-14868D | 10,0 |

Die Strukturformel des Antibiotikums X-14868B ist wie folgt:

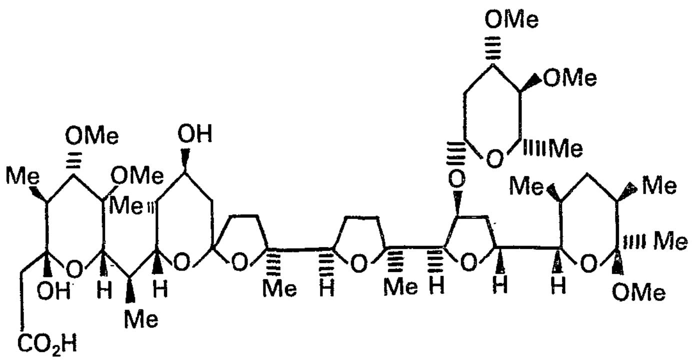

II

Die Infrarot-Absorptionsspektra für die oben beschriebenen Verbindungen sind wie folgt:

| | |
|---|---|
| X-14868A | – Figur 1 |
| X-14868B | – Figur 2 |
| X-14868C | – Figur 3 |
| X-14868D | – Figur 4 |

In der nachstehenden Tabelle 9 sind die physikalischen Konstanten für die oben erwähnten Substanzen zusammengestellt.

Tabelle 9
Physikalische Konstanten für X-14868-Substanzen

| Substanznummer | Schmelzpunkt, °C | [α]D, spezifische Drehung Chloroform | Methanol | %C | * Mikroanalyse; gefunden: %H | %N | %OMe |
|---|---|---|---|---|---|---|---|
| X-14868A | 193–195 | +40.6° | +23.8° | 60.11 | 8.54 | 2.38 | 12.10 |
| X-14868B | 172.5–174 | +46.1° | +34.8° | 60.97 | 8.60 | 1.23 | 14.87 |
| X-14868C | 172–175 | +49.6° | +30.5° | 57.13 | 8.58 | 2.36 | 10.25 |
| X-14868D | 194–195 | +41.1° | +29.2° | 60.32 | 8.80 | 3.40 | |

| *Berechnete Mikroanalyse für Substanzen A und B | Mol. gew. | %C | %H | %Na | %OMe |
|---|---|---|---|---|---|
| X-14868A: $C_{47}H_{79}O_{17}Na$ | 939.25 | 60.10 | 8.50 | 2.45 | 13.22 |
| X-14868B: $C_{96}H_{163}O_{34}Na.H_2O$ | 1902.36 | 60.61 | 8.75 | 1.21 | 16.54 |

Die nachstehenden Beispiele veranschaulichen die Erfindung.

Beispiel 1

Schüttelkolbengärung mit Nocardia X-14868

Die das Antibiotikum X-14868A erzeugende Kultur wird auf einer Stärke-Caseinagarschrägkultur folgender Zudammensetzung (g/l in destilliertem Wasser) gezüchtet und aufbewahrt:

| | |
|---|---|
| Lösliche Stärke | 10,0 |
| Casein | 1,0 |
| $K_2HPO_4$ | 0,5 |
| $MgSO_4$ (wasserfrei) | 0,5 |
| Agar | 20,0 |

Der pH-Wert wird auf 7,4 mit wässriger Natriumhydroxidlösung eingestellt. Anschliessend wird die Kultur bei 1 kg/cm² 20 Minuten autoklaviert.

Die Schrägkultur wird mit der Nocardia X-14868-Kultur beimpft und bei 20 °C 7–14 Tage bebrütet. Ein Teil des Agars mit Mycel aus der gut wachsenden Agarkultur wird zur Beimpfung eines 500 ml Erlenmeyerkolbens mit 100 ml sterilisiertem Impfsubstrat folgender Zusammensetzung (g/l in destilliertem Wasser) verwendet:

| | |
|---|---|
| Tomatenmark | 5,0 |
| getrocknete Rückstände aus der Brennerei | 5,0 |
| Pepton | 5,0 |
| entbitterte getrocknete Hefe | 5,0 |
| Maisstärke | 20,0 |
| $CaCO_3$ | 1,0 |
| $K_2HPO_4$ | 1,0 |

Der pH-Wert wird vor der Sterilisierung mit wässriger Natriumhydroxidlösung auf 7 eingestellt.

Die beimpfte Nährlösung wird bei 28 °C 72 Stunden auf einer Schüttelmaschine (250 upm) bebrütet.

Eine 3 ml Portion (3% v/v) der erhaltenen Kultur wird zur Beimpfung eines 500 ml Erlenmeyerkolbens mit 100 ml sterilisiertem Nährmedium folgender Zusammensetzung (g/l in destilliertem Wasser) verwendet:

| | |
|---|---|
| Glycerin | 40,0 |
| Pepton | 5,0 |
| Cerelose | 2,0 |
| Kartoffelstärke | 2,0 |
| entfetteter Soyagriess | 5,0 |
| entbitterte getrocknete Hefe | 5,0 |
| NaCl | 5,0 |
| $CaCO_3$ | 0,2 |

Der pH-Wert wird vor dem Autoklavieren mit wässriger Natriumhydroxidlösung auf 6,4 eingestellt.

Die beimpfte Nährlösung wird bei 28 °C 5 Tage auf einer Schüttelmaschine (250 upm) bebrütet. Der Gehalt an Antibiotikum X-14868A in der Gärbrühe wird durch Analyse mit Staphylococcus aureus ATCC 6538P mit Hilfe von Agardiffusion/Lochplattentechnik bestimmt.

Beispiel 2

Die Isolierung von Antibiotikum X-14868A Natriumsalz und Antibiotikum X-14868B Natriumsalz aus Schüttelkolbengärungen.

Stufe A: Die ganze Gärlösung aus 50 500 ml Erlenmeyerkolben mit je 100 ml Gärbrühe wird nach 5 Tagen Gärung vereinigt (5 Liter) und zweimal mit der halben Menge Äthylacetat extrahiert. Nach halbstündigem Rühren wird die Lösungsmittelschicht abgetrennt und unter vermindertem Druck zu einem Öl (4 g) konzentriert. Das Öl wird in Diäthyläther aufgelöst und an einer 200 g Kieselgelkolonne (durch Aufschlämmung in Diäthyläther erstellt) chromatographiert. Die Kolonne wird mit einem Gradienten zwischen 2 Liter Diäthyläther bis 2 Liter Diäthyläther/Aceton (9:1) eluiert, anschliessend mit 2 Liter Diäthyläther/Aceton (1:1) und schliesslich mit 2 Liter Methylenchlorid/Äthylalkohol (7:3). 40 ml-Fraktionen werden gesammelt und die Fraktionen Nr. 18–75 werden vereinigt, das Lösungsmittel wird unter vermindertem Druck entfernt und der so erhaltene Rückstand (1,66 g) in Äthylacetat aufgelöst, mit einer gleichen Menge 1N wässriger Salzsäure zweimal gewaschen und anschliessend mit einer gleichen Menge gesättigter Natriumcarbonatlösung zweimal gewaschen. Die Lösungsmittelphase wird über Natriumsulfat getrocknet. Durch Zugabe von n-Hexan erhält man das Antibiotikum X-14868A-Natriumsalz als Kristalle. Umkristallisieren aus Äthylacetat/n-Hexan liefert eine analytische Probe des Antibiotikums X-14868A-Natriumsalz, Schmelzpunkt 193–194 °C.

Stufe B: Die Fraktionen Nr. 161–215 werden vereinigt, das Lösungsmittel unter vermindertem Druck entfernt und der Rückstand in Äthylacetat gelöst, mit 1N wässriger Salzsäure gewaschen und anschliessend mit wässriger gesättigter Natriumcarbonatlösung gewaschen, über Natriumsulfat getrocknet und mit n-Hexan versetzt. Man erhielt in dieser Weise Antibiotikum X-14868B-Natriumsalz als Kristalle, welche bei 172,5–174 °C schmelzen.

Beispiel 3

Tankgärung mit Nocardia X-14868

Die das Antibiotikum X-14868A erzeugende Kultur Nocardia X-14868 wird auf einer Stärke-Caseinagarschrägkultur folgender Zusammensetzung (g/l destilliertes Wasser) gezüchtet und aufbewahrt:

| | |
|---|---|
| Lösliche Stärke | 10,0 |
| Casein | 1,0 |
| $K_2HPO_4$ | 0,5 |
| $MgSO_4$ (wasserfrei) | 0,5 |
| Agar | 20,0 |

Der pH-Wert wird vor dem Autoklavieren mit wässriger Natriumhydroxidlösung auf 7,4 eingestellt.

Die Schrägkultur wird mit der Nocardia X-14868-Kultur beimpft und bei 28 °C 7–14 Tage bebrütet. Ein Teil des Agars mit Mycel aus dem gut wachsenden Agar wird anschliessend verwendet zur Herstellung eines vegetativen Inoculums durch Beimpfung eines 500 ml Erlenmeyerkolbens mit 100 ml Nährmedium folgender Zusammensetzung (g/l destilliertes Wasser):

| | |
|---|---|
| Tomatenmark | 5,0 |
| getrocknete Rückstände aus der Brennerei | 5,0 |
| Pepton | 5,0 |
| entbitterte getrocknete Hefe | 5,0 |
| Maisstärke | 20,0 |
| $CaCO_3$ | 1,0 |
| $K_2HPO_4$ | 1,0 |

Der pH-Wert wird vor dem Autoklavieren auf 7,0 eingestellt.

Die beimpfte Nährlösung wird 72 Stunden bei 28 °C auf einer Schüttelmaschine (250 upm) bebrütet.

60 ml (3% v/v) dieser Kulturlösung wird zur Beimpfung eines 6 l Erlenmeyerkolbens mit 2 l Impfmedium folgender Zusammensetzung (g/l destilliertes Wasser) verwendet:

| | |
|---|---|
| Tomatenmark | 5,0 |
| getrocknete Rückstände aus der Brennerei | 5,0 |
| Pepton | 5,0 |
| entbitterte getrocknete Hefe | 5,0 |
| Maisstärke | 20,0 |
| $CaCO_3$ | 1,0 |
| $K_2HPO_4$ | 1,0 |

Der pH-Wert wird vor dem Autoklavieren auf 7,0 eingestellt.

Das beimpfte Nährmedium wird 72 Stunden bei 28 °C auf einer Schüttelmaschine (250 upm) bebrütet.

Eine 4 l Portion der erhaltenen Kultur wird zur Beimpfung von 230 l Nährmedium folgender Zusammensetzung (g/l Leitungswasser) in einem 380 l Fermenter verwendet:

| | |
|---|---|
| Glycerin | 40,0 |
| Pepton | 5,0 |
| Cerelose | 2,0 |
| Kartoffelstärke | 2,0 |
| entfetteter Soyagriess | 5,0 |
| entbitterte getrocknete Hefe | 5,0 |
| NaCl | 5,0 |
| $CaCO_3$ | 0,2 |
| Schaumbekämpfungsmittel | 0,1 |

Der pH-Wert des Mediums wird auf 6,4 eingestellt. Anschliessend wird 1¼ Stunden mit Dampf von 4,2 kg/cm$^2$ sterilisiert.

Das beimpfte Medium wird mit Druckluft von einer Geschwindigkeit von 90 l pro Minute belüftet und mit einem Rührwerk (280 upm) gerührt. Die Gärung wird bei 28 °C 5 Tage durchgeführt.

Der Gehalt an Antibiotikum X-14868A in der Gärlösung wird durch Analyse mittels Staphylococcus aureus ATCC 6538P unter Verwendung von Agardiffusion/Lochplattentechnik bestimmt.

Beispiel 4

Isolierung der Antibiotika X-14868A, C und D (Natriumsalze) aus Tankgärungen von Nocardia X-14868.

Stufe A: Die Gärlösung aus 230 l Gärung gemäss Beispiel 3 wird nach 115-stündiger Gärungszeit mit der gleichen Menge Äthylacetat versetzt. Nach einstündigem Rühren wird die Lösungsmittelschicht abgetrennt und unter vermindertem Druck auf 4,2 l eingedampft. Der konzentrierte Extrakt wird zweimal mit der gleichen Menge 1N wässriger Salzsäure gewaschen, gefolgt von zweimaligem Waschen mit der gleichen Menge gesättigter wässriger Natriumcarbonatlösung. Die Lösungsmittelphase wird über Natriumsulfat getrocknet und unter vermindertem Druck zu einem Öl konzentriert. Das erhaltene Öl wird in n-Hexan gelöst und mti Acetonitril extrahiert, gefolgt von zwei Extraktionen mit Acetonitril/Methanol (8:2). Die Acetonitril- und Acetonitril/Methanol (8:2)-Extrakte werden vereinigt und das Lösungsmittel wird unter vermindertem Druck entfernt. Das erhaltene Öl wird in Diäthyläther gelöst, mit Entfärbungskohle behandelt und filtriert. Nach Zugabe von n-Hexan erhält man rohes kristallines Natriumsalz des Antibiotikums X-14868A. Dieses wird in Methylenchlorid gelöst und an einer 600 g Kieselgelkolonne chromatographiert. Die Kolonne wird mit 4 l Diäthyläther/Aceton/Ammoniumhydroxid (8/2/0,02) eluiert. 45-ml-Fraktionen werden gesammelt; die Fraktionen 13–15 werden vereinigt. Das Lösungsmittel wird unter vermindertem Druck entfernt, der Rückstand in Äthylacetat gelöst und nacheinander gewaschen, zweimal mit 1N wässriger Salzsäure und zweimal mit wässriger, gesättigter Natriumcarbonatlösung und anschliessend über Natriumsulfat getrocknet. Die Äthylacetatphase wird zu einem Öl konzentriert, welches in Methylenchlorid gelöst wird. Nach Zugabe von n-Hexan erhält man kristallines Natriumsalz des Antibiotikums X-14868A vom Schmelzpunkt 193–195 °C.

Stufe B: Die Mutterlauge der rohen Kristalle des Natriumsalzes vom Antibiotikum X-14868A (erhalten gemäss obiger Stufe A) wird an einer 1 kg Kieselgelkolonne chromatographiert. Die Kolonne wird mit 1 l n-Hexan eluiert und anschliessend mit einem Gradienten zwischen 4 l Diäthyläther/n-Hexan (7:3) bis 4 l Diäthyläther/Aceton (8:2). 40-ml-Fraktionen werden gesammelt; die Fraktionen 43–80 werden vereinigt. Das Lösungsmittel wird unter vermindertem Druck entfernt und der Rückstand in Äthylacetat gelöst und nacheinander mit 1N wässriger Salzsäure und wässriger gesättigter Natriumcarbonatlösung gewaschen. Nach Behandlung mit Entfärbungskohle und Filtration wird die Äthylacetatphase zu einem Öl konzentriert. Dieses Öl wird in Diäthyläther gelöst. Nach Zugabe von n-Hexan erhält man eine zusätzliche Menge Natriumsalz des Antibiotikums X-14868A vom Schmelzpunkt 193–195 °C.

Stufe C: Fraktionen 22–40 aus der oben in Stufe A beschriebenen Kieselgelkolonne liefern kristallines Natriumsalz des Antibiotikums X-14868C vom Schmelzpunkt 172–175 °C.

Stufe D: Die Mutterlauge des in Stufe B beschriebenen kristallinen Natriumsalzes des Antibiotikums X-14868A und die Fraktionen 16–21 aus der in Stufe A beschriebenen Kieselgelkolonne werden vereinigt, konzentriert und an einer 600 g Kieselgelkolonne chromatographiert. Die Kolonne wird mit 4 l Diäthyläther/Hexan (1:1), 4 l Diäthyläther / Aceton / n-Hexan / Ammoniumhydroxid (6:2:2:0,002); 2 l Diäthyläther/Aceton (8:2) eluiert. 40-ml-Fraktionen werden gesammelt. Die Fraktionen 71–98 werden vereinigt; Kristallisation liefert eine zusätzliche Menge kristallines Natriumsalz des Antibiotikums X-14868A. Die Fraktionen 141–172 werden ebenfalls vereinigt und das Lösungsmittel unter vermindertem Druck entfernt. Kristallisieren aus Diäthyläther/n-Hexan liefert das Natriumsalz des Antibiotikums X-14868D vom Schmelzpunkt 194–195 °C.

Beispiel 5

Herstellung des Thalliumsalzes des Antibiotikums X-14868A.

Eine Lösung von 51 mg Natriumsalz des Antibiotikums X-14868A in Methylenchlorid wird gewaschen (nacheinander mit 1N wässriger Salzsäure, Wasser und anschliessend viermal mit einer wässrigen Lösung von Thalliumcarbonat). Das Lösungsmittel wird abgetrennt und unter vermindertem Druck auf ein kleines Volumen konzentriert. Nach Zugabe von n-Hexan erhält man kristallines Thalliumsalz des Antibiotikums X-14868A. Umkristallisation aus Diäthyläther/n-Hexan liefert für Röntgenanalyse geeignete Kristalle.

Beispiel 6

Herstellung des Calciumsalzes des Antibiotikums X-14868A.

Eine Lösung von 200 mg Natriumsalz des Antibiotikums X-14868A in Äthylacetat wird nacheinander mit 1N wässriger Salzsäure und dreimal mit gesättigter wässriger Calciumhydroxidlösung gewaschen. Das Lösungsmittel wird abgetrennt und unter vermindertem Druck entfernt. Man erhält das Calciumsalz des Antibiotikums X-14868A als einen weissen, festen Schaum.

Beispiel 7

Schüttelkolbengärung mit Nocardia X-14868

Der Mikroorganismus X-14868 wird auf einer Stärke-Caseinagarschrägkultur folgender Zusammensetzung (g/l in destilliertem Wasser) gezüchtet und aufbewahrt:

| | |
|---|---|
| Lösliche Stärke | 10,0 |
| Casein | 1,0 |
| $K_2HPO_4$ | 0,5 |
| $MgSO_4$ (wasserfrei) | 0,5 |
| Agar | 20,0 |

Der pH-Wert wird mit wässriger Natriumhydroxidlösung auf 7,4 eingestellt; anschliessend wird 20 Minuten bei 1 kg/cm$^2$ autoklaviert.

Die Schrägkultur wird mit der Nocardia X-14868-Kultur beimpft und bei 28 °C 7–14 Tage bebrütet. Ein Teil des Agars mit Mycel aus gut wachsendem Agar wird zur Beimpfung eines 500 ml Erlenmeyerkolbens mit 100 ml sterilisiertem Impfsubstrat folgender Zusammensetzung (g/l in destilliertem Wasser) verwendet:

| | |
|---|---|
| Tomatenmark | 5,0 |
| getrocknete Rückstände aus der Brennerei | 5,0 |
| Pepton | 5,0 |
| entbitterte getrocknete Hefe | 5,0 |
| Maisstärke | 20,0 |
| $CaCO_3$ | 1,0 |
| $K_2HPO_4$ | 1,0 |

Der pH-Wert wird vor der Sterilisierung mit wässriger Natriumhydroxidlösung auf 7 eingestellt.

Die beimpfte Nährlösung wird bei 28 °C 72 Stunden auf einer Schüttelmaschine (250 upm) bebrütet.

Eine 3 ml Portion (3% v/v) der erhaltenen Kultur wird zur Beimpfung eines 500 ml Erlenmeyerkolbens mit 100 ml sterilisiertem Nährmedium folgender Zusammensetzung (g/l in destilliertem Wasser) verwendet:

| | |
|---|---|
| Cerelose | 45,0 |
| Stärke | 20,0 |
| Soyabohnenmehl | 15,0 |
| Caseinhydrolysat (sauer) | 1,0 |
| Melasse | 3,0 |
| $CaCO_3$ | 2,5 |
| Leitungswasser bis 1 Liter | |
| pH 7,1 | |

Die beimpfte Nährlösung wird bei 28 °C 7 Tage auf einer Schüttelmaschine (250 upm) bebrütet. Der Gehalt an Antibiotikum X-14868A in der Gärlösung wird durch Analyse mit Staphylococcus aureus ATCC 6538P unter Verwendung von Agardiffusion/Lochplattentechnik bestimmt.

**Patentansprüche für die Vertragsstaaten: BE, CH, DE, FR, GB, IT, LI, LU, NL, SE,**

1. Polyätherverbindungen, d.h. die Antibiotika X-14868A und X-14868B der allgemeinen Formel

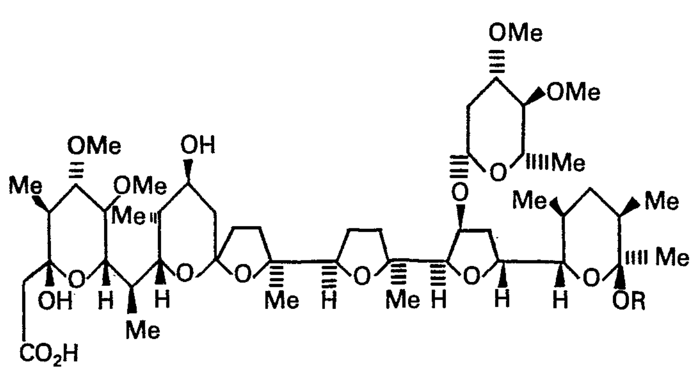

I

worin R Wasserstoff oder Methyl darstellt, und ihre pharmazeutisch verträglichen Salze.

2. Ver~~b~~indungen gemäss Anspruch 1, dadurch gekennzeichnet, dass R Wasserstoff darstellt, d.h. Antibiotikum X-14868A und seine pharmazeutisch verträglichen Salze.

3. Antibiotikum X-14868C und seine pharmazeutisch verträglichen Salze, wovon das Natriumsalz folgende physikalische Eigenschaften besitzt:
1) Ein Infrarotspektrum gemäss Fig. 3;
2) einen Schmelzpunkt von 172–175 °C;
3) eine spezifische Drehung von

| [α]D Chloroform | Methanol |
|---|---|
| +49,6° | +30,5° und |

4) eine Mikroanalyse von

| %C | %H | %Na |
|---|---|---|
| 57,13 | 8,58 | 2,36 |

4. Antibiotikum X-14868D und seine pharmazeutisch verträglichen Salze, wovon das Natriumsalz folgende physikalische Eigenschaften besitzt:
1) Ein Infrarotspektrum gemäss Fig. 4;
2) einen Schmelzpunkt von 194–195 °C;
3) eine spezifische Drehung von

| [α]D Chloroform | Methanol |
|---|---|
| +41,1 ° | +29,2 ° und |

4) eine Mikroanalyse von

| %C | %H | %Na |
|---|---|---|
| 60,32 | 8,80 | 3,40 |

5. Verbindungen gemäss einem der Ansprüche 1–4 als pharmazeutische Wirkstoffe.

6. Verbindungen gemäss einem der Ansprüche 1–4 als pharmazeutische Wirkstoffe zur Behandlung und Prophylaxe von bakteriellen Krankheiten und/oder Coccidiose.

7. Verbindungen gemäss Anspruch 2 als pharmazeutische Wirkstoffe zur Behandlung und Prophylaxe von Schweinedysenterie.

8. Verbindungen gemäss Anspruch 2 als wachstumsfördernde Mittel bei Wiederkäuern.

9. Verfahren zur Herstellung der Polyätherverbindungen gemäss einem der Ansprüche 1–4, dadurch gekennzeichnet, dass man eine Subkultur des Stammes Nocardia X-14868 ATCC 31585 in einer Kohlenhydrate und stickstoffhaltige Nährstoffe enthaltenden wässrigen Lösung unter submersen aeroben Bedingungen kultiviert und anschliessend die Endprodukte aus der Lösung abtrennt und isoliert.

10. Pharmazeutische Präparate, gekennzeichnet durch einen Gehalt an einer Verbindung gemäss einem der Ansprüche 1–4.

11. Pharmazeutische Präparate zur Behandlung und Prophylaxe von bakteriellen Krankheiten und/oder Coccidiose, gekennzeichnet durch einen Gehalt an einer Verbindung gemäss einem der Ansprüche 1–4.

12. Pharmazeutische Präparate zur Behandlung und Prophylaxe von Schweinedysenterie, gekennzeichnet durch einen Gehalt an einer Verbindung gemäss Anspruch 2.

13. Vormischung oder Komposition zum unmittelbaren Verbrauch als wachstumsförderndes Mittel bei Wiederkäuern, enthaltend eine Verbindung gemäss Anspruch 2.

**Patentansprüche für den Vertragsstaat: AT**

1. Verfahren zur Herstellung von Polyätherverbindungen, dadurch gekennzeichnet, dass man eine Subkultur des Stammes Nocardia X-14868 ATCC 31585 in einer Kohlenhydrate und stickstoffhaltige Nährstoffe enthaltenden wässrigen Lösung unter submersen aeroben Bedingungen kultiviert und anschliessend die Endprodukte aus der Lösung abtrennt und isoliert.

2. Verfahren nach Anspruch 1, dadurch gekennzeichnet, dass man als Endprodukt das Antibiotikum X-14868A der Formel

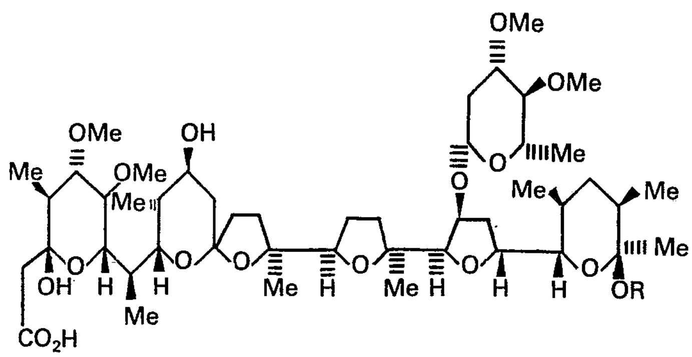

I

worin R Wasserstoff darstellt,

oder ein pharmazeutisch verträgliches Salz dieser Verbindung isoliert.

3. Verfahren nach Anspruch 1, dadurch gekennzeichnet, dass man als Endprodukt das Antibiotikum X-14868B der Formel

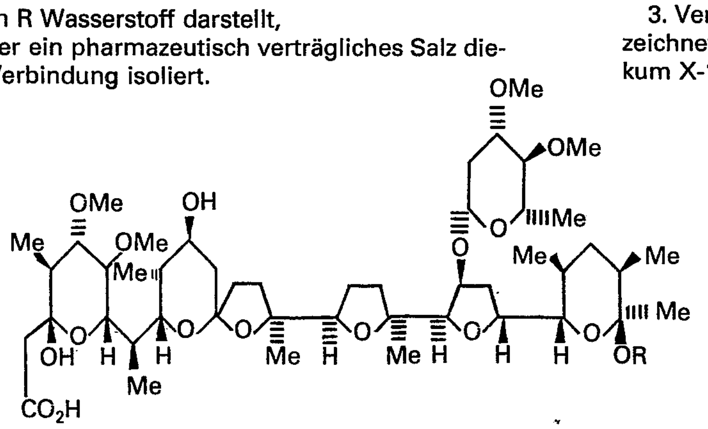

I

worin R Methvl darstellt, oder ein pharmazeutisch verträgliches Salz dieser Verbindung isoliert.

4. Verfahren nach Anspruch 1, dadurch gekennzeichnet, dass man als Endprodukt das Antibiotikum X-14868C, welches als Natriumsalz folgende physikalische Eigenschaften besitzt:

1) Ein Infrarotspektrum gemäss Fig. 3;
2) einen Schmelzpunkt von 172–175 °C;
3) eine spezifische Drehung von
[α]D Chloroform Methanol
+49,6° +30,5° und
4) eine Mikroanalyse von

| %C | %H | %Na |
|---|---|---|
| 57,13 | 8,58 | 2,36 |

oder ein pharmazeutisch verträgliches Salz dieser Verbindung isoliert.

5. Verfahren nach Anspruch 1, dadurch gekennzeichnet, dass man als Endprodukt das Antibiotikum X-14868D, welches als Natriumsalz folgende physikalische Eigenschaften besitzt:

1) Ein Infrarotspektrum gemäss Fig. 4;
2) einen Schmelzpunkt von 194–195°;
3) eine spezifische Drehung von
[α] D Chloroform Methanol
+41,1° +29,2° und
4) eine Mikroanalyse von

| %C | %H | %N |
|---|---|---|
| 60,32 | 8,80 | 3,40 |

oder ein pharmazeutisch verträgliches Salz dieser Verbindung isoliert.

**Revendications pour les Etats: BE, CH, LI, DE, FR, GB, IT, LU, NL, SE,**

1. Composés polyéthers, à savoir les antibiotiques X-14868A et X-14868B de formule générale:

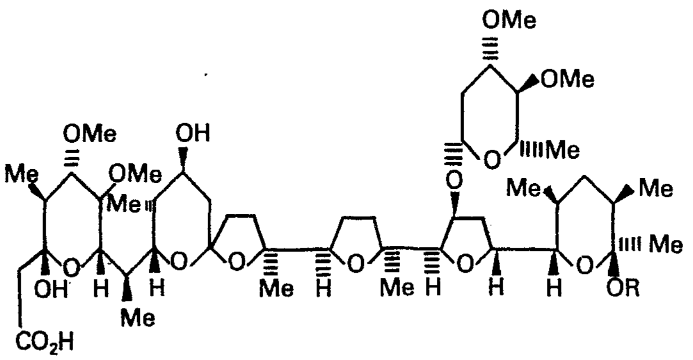

I

où R représente un hydrogène ou un méthyle, et leurs sels pharmaceutiquement acceptables.

2. Composés selon la revendication 1, caractérisés en ce que R représente un hydrogène, c'est-à-dire l'antibiotique X-14868A et ses sels pharmaceutiquement acceptables.

3. Antibiotique X-14868C et ses sels pharmaceutiquement acceptables, dont le sel de sodium possède les propriétés physiques suivantes:

1) un spectre infrarouge selon la Figure 3;
2) un point de fusion de 172–175 °C;
3) un pouvoir rotatoire spécifique de
[α]D chloroforme Méthanol
+49,6° +30,5° et
4) des valeurs de microanalyse de

| %C | %H | %Na |
|---|---|---|
| 57,13 | 8,58 | 2,36 |

4. Antibiotique X-14848D et ses sels pharmaceutiquement acceptables, dont le sel de sodium possède les propriétés physiques suivantes:

1) un spectre infrarouge selon la Figure 4;
2) un $P_f$ de 194–195°:
3) un pouvoir rotatoire spécifique de
[α]D chloroforme Méthanol
+41,1° +29,2° et
4) une valeur de microanalyse de

| %C | %H | %Na |
|---|---|---|
| 60,32 | 8,80 | 3,40 |

5. Composés selon l'une des revendications 1 à 4 comme substances actives pharmaceutiques.

6. Composés selon l'une des revendications 1 à 4 comme substances pharmaceutiques pour le traitement et la prophylaxie des maladies bactériennes et/ou de la coccidiose.

7. Composés selon la revendication 2 comme substances actives pharmaceutiques pour le traitement et la prophylaxie de la dysenterie du porc.

8. Composés selon la revendication 2 comme agents favorisant la croissance chez les ruminants.

9. Procédé de préparation des composés polyéthers selon l'une des revendications 1 à 4, caractérisé en ce qu'on cultive une sous-culture de la souche Nocardia X-14868 ATCC 31585 dans une solution aqueuse contenant des hydrates de carbone et des produits nutritifs azotés dans des conditions aérobies en gélose profonde puis en ce qu'on sépare et on isole les produits finals de la solution.

10. Préparations pharmaceutiques caractérisées en ce qu'elles contiennent un composé selon l'une des revendications 1–4.

11. Préparations pharmaceutiques pour le traitement et la prophylaxie des maladies bactériennes et/ou de la coccidiose, caractérisées en ce qu'elles contienent un composé selon l'une des revendications 1–4.

12. Préparations pharmaceutiques pour le traitement et la prophylaxie de la dysentrie du porc, caractérisées en ce qu'elles contiennent un composé selon la revendication 2.

13. Prémélange ou composition pour utilisation immédiate comme agent favorisant la croissance chez les ruminants, contenant un composé selon la revendication 2.

**Revendications pour l'Etat: AT**

1. Procédé de préparation de composés polyéthers, caractérisé en ce qu'on cultive une sousculture de la souche Nocardia X-14868 ATCC 31585 dans une solution aqueuse contenant des hydrates de carbone et des produits nutritifs azotés dans des conditions aérobies en gélose profonde puis en ce qu'on sépare et isole les produits finals de la solution.

2. Procédé selon la revendication 1, caractérisé en ce qu'on isole comme produit final l'antibiotique X-14868A de formule

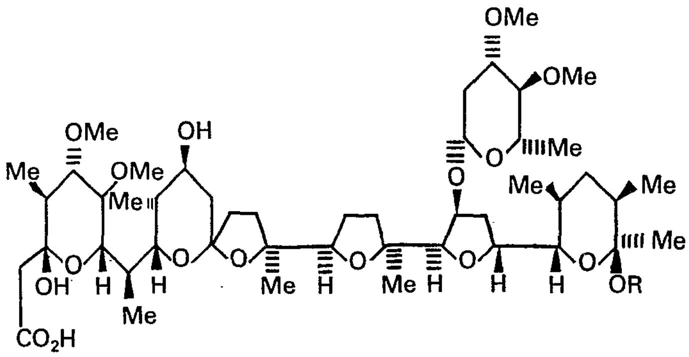

I

où R représente un hydrogène, ou un sel pharmaceutiquement compatible de ce composé.

3. Procédé selon la revendication 1, caractérisé en ce qu'on isole comme produit final l'antibiotique X-14868B de formule:

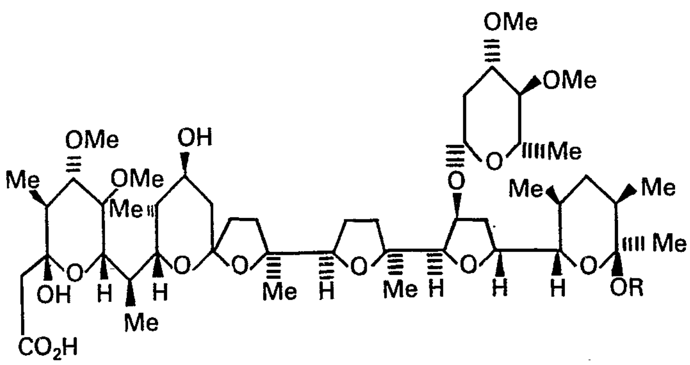

I

où R représente un méthyle, ou un sel pharmaceutiquement acceptable de ce composé.

4. Procédé selon la revendication 1, caractérisé en ce qu'on isole ocmme produit final l'antibiotique X-14868C, qui comme sel de sodium possède les propriétés physiques suivantes:

1) un spectre infrarouge selon la Figure 3;
2) un point de fusion de 172–175 °C,
3) un pouvoir rotatoire spécifique de

| [α]D chloroforme | Méthanol |
|---|---|
| +49,6° | +30,5° et |

4) une valeur de microanalyse de

| %C | %H | %Na |
|---|---|---|
| 57,13 | 8,58 | 2,36 |

ou un sel pharmaceutiquement acceptable de ce composé.

5. Procédé selon la revendication 1, caractérisé en ce qu'on isole comme produit final l'antibiotique X-14868D, qui possède comme sel de sodium les propriétés physiques suivantes:

1) un spectre infrarouge selon la Figure 4,
2) un $P_f$ de 194–195°;
3) un pouvoir rotatoire spécifique de

| [α]D chloroforme | Méthanol |
|---|---|
| +41,1° | +29,2° et |

4) une valeur de microanalyse de

| %C | %H | %Na |
|---|---|---|
| 60,32 | 8,80 | 3,40 |

ou un sel pharmaceutiquement acceptable de ce composé.

**Claims for the contracting States: BE, CH, LI, DE, FR, GB, IT, LU, NL, SE,**

1. Polyether compounds, i.e. the antibiotics X-14868A and X-14868B of the general formula

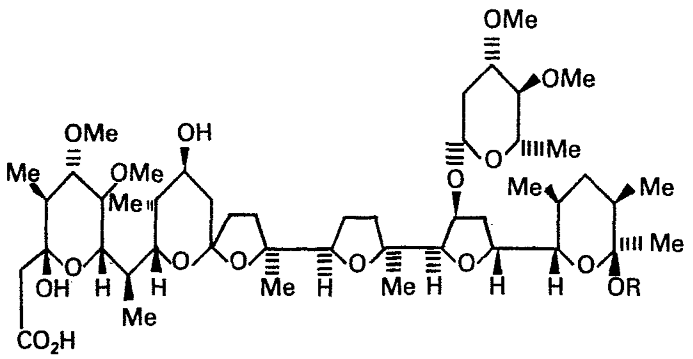

I

wherein R represents hydrogen or methyl, and their pharmaceutically acceptable salts.

2. Compounds in accordance with claim 1, characterized in that R represents hydrogen, i.e.antibiotic X-14868A and its pharmaceutically acceptable salts.

3. Antibiotic X-14868C and its pharmaceutically acceptable salts, of which the sodium salt has the following physical characteristics:

1) An infrared spectrum in accordance with Fig. 3;
2) a melting point of 172–175 °C;
3) a specific rotation of

| [α]D chloroform | methanol |
|---|---|
| +49,6° | +30,5° and |

4) a microanalysis of

| %C | %H | %Na |
|---|---|---|
| 57.13 | 8.58 | 2.36 |

4. Antibiotic X-14868D and its pharmaceutically acceptable salts, of which the sodium salt has the following physical characteristics:

1) An infrared spectrum in accordance with Fig. 4;
2) a melting point of 194–195°;
3) a specific rotation of

| [α]D chloroform | methanol |
|---|---|
| +41.1° | +29.2° and |

4) a microanalysis of

| %C | %H | %Na |
|---|---|---|
| 60.32 | 8.80 | 3.40 |

5. Compounds according to any one of claims 1–4 as pharmaceutically active substances.

6. Compounds according to any one of claims 1–4 as pharmaceutically active substances for the treatment and prophylaxis of bacterial diseases and/or coccidiosis.

7. Compounds according to claim 2 as pharmaceutically active substances for the treatment and prophylaxis of swine dysentery.

8. Compounds according to claim 2 as growth promoting agents in ruminants.

9. Process for the manufacture of the polyether compounds according to any one of claims 1–4, characterized by cultivating a subculture of the strain Nocardia X-14868 ATCC 31585 in an aqueous solution containing carbohydrate and nitrogen-containing nutrient substances under submersed aerobic conditions and subsequently separating and isolating the end products from the solution.

10. Pharmaceutical preparation, characterized by a content of a compound in accordance with any one of claims 1–4.

11. Pharmaceutical preparation for the treatment and prophylaxis of bacterial diseases and/or coccidiosis, characterized by a content of a compound in accordance with any one of claims 1–4.

12. Pharmaceutical preparation for the treatment and prophylaxis of swine dysentery, characterized by a content of a compound in accordance with claim 2.

13. Premix or composition for the ready consumption as a growth promoting agent in ruminants, containing a compound in accordance with claim 2.

**Claims for contracting state AT**

1. Process for the manufacture of polyether compounds, characterized by cultivating a subculture of the strain Nocardia X-14868 ATCC 31585 in an aqueous solution containing carbohydrate and nitrogen-containing nutrient substances under submersed aerobic conditions and subsequently separating and isolating the end products from the solution.

2. Process according to claim 1, characterized in that the antibiotic X-14868A of the formula

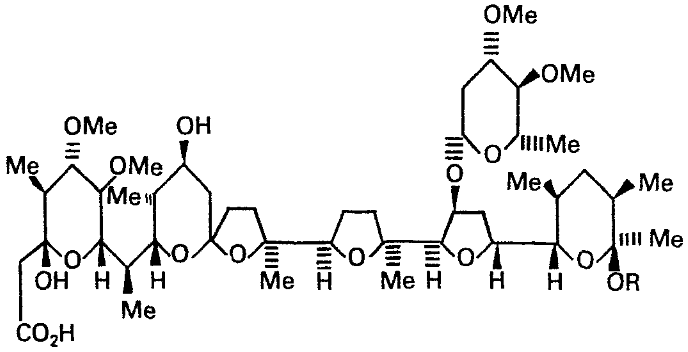

I

wherein R represents hydrogen,
or a pharmaceutically acceptable salt of this compound is isolated as the end product.

3. Process according to claim 1, characterized in that the antibiotic X-14868B of the formula

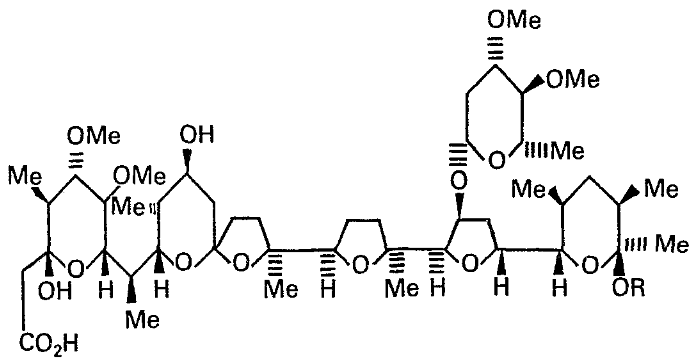

I

wherein R represents methyl, or a pharmaceutically acceptable salt of this compound is isolated as the end product.

4. Process according to claim 1, characterized in that the antibiotic X-14868C, which as the sodium salt has the following physical characteristics:

1) An infrared spectrum in accordance with Fig. 3;
2) a melting point of 172–175 °C;
3) a specific rotation of

| [α]D chloroform | methanol |
|---|---|
| +49.6° | +30.5°and |

4) a microanalysis of

| %C | %H | %Na |
|---|---|---|
| 57.13 | 8.58 | 2.36 |

or a pharmaceutically acceptable salt of this compound is isolated as the end product.

5. Process according to claim 1, characterized in that the antibiotic X-14868D, which as the sodium salt has the following physical characteristics:

1) An infrared spectrum in accordance with Fig. 4;
2) a metling poing of 194–195°;
3) a specific rotation of

| [α]D chloroform | methanol |
|---|---|
| +41.1° | +29.2° and |

4) a microanalysis of

| %C | %H | %Na |
|---|---|---|
| 60.32 | 8.80 | 3.40 |

or a pharmaceutically acceptable salt of this compound is isolated as the end product.

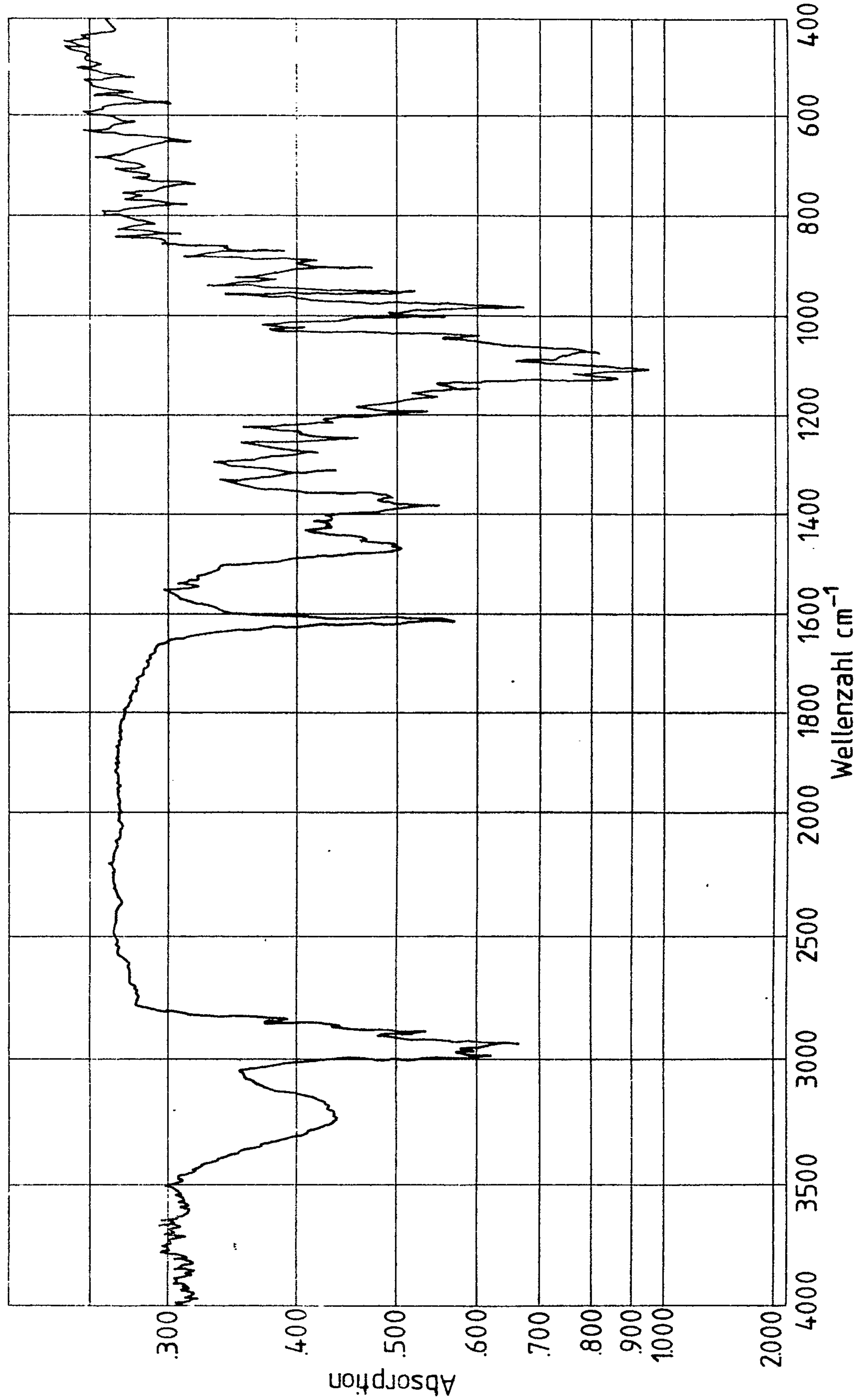
Absorption
.300
.400
.500
.600
.700
.800
.900
1.000
2.000
4000
3500
3000
2500
2000
1800
1600
1400
1200
1000
800
600
400
Wellenzahl cm⁻¹

FIG.1

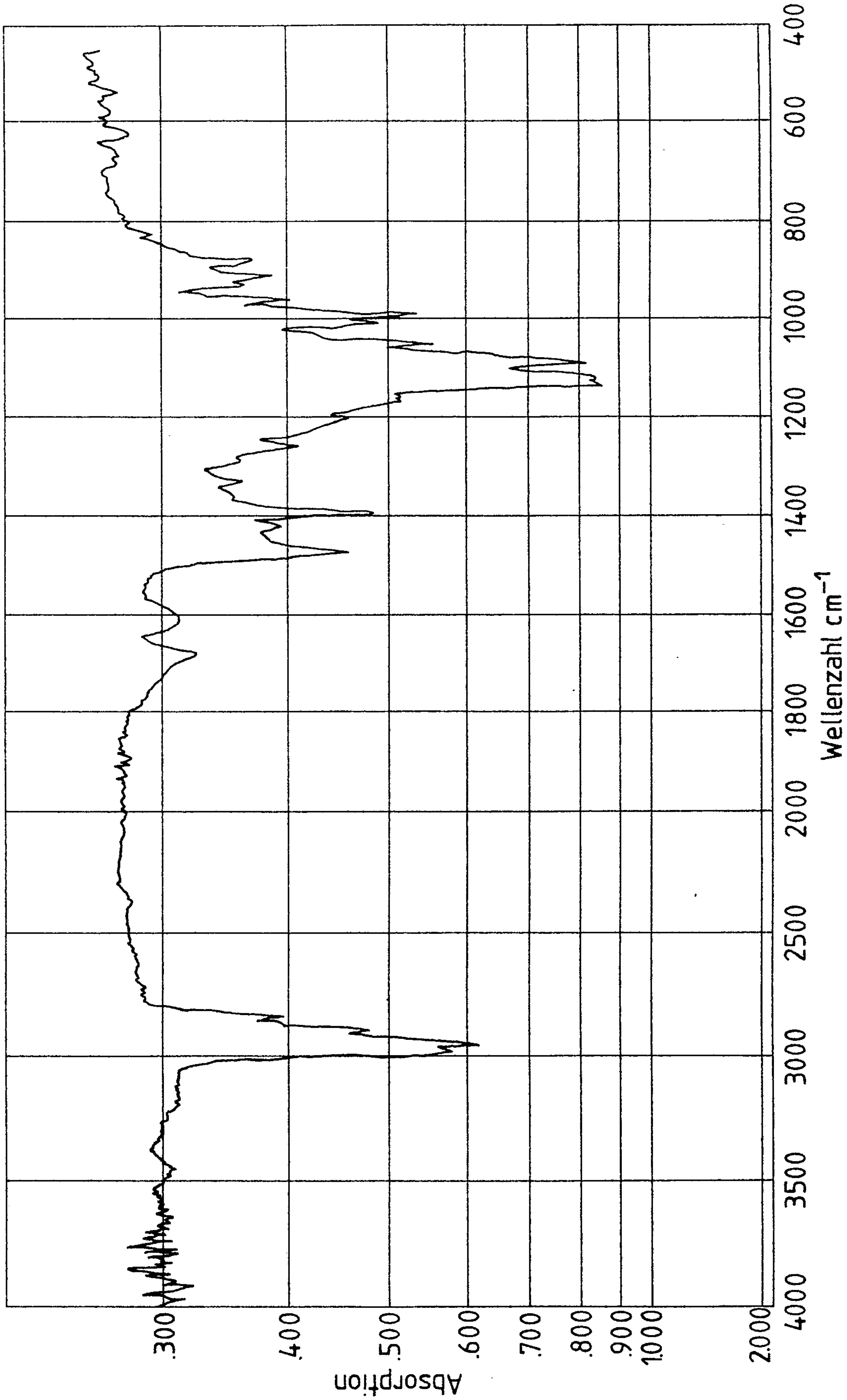
Absorption
.300
.400
.500
.600
.700
.800
.900
1.000
2.000
4000
3500
3000
2500
2000
1800
1600
1400
1200
1000
800
600
400
Wellenzahl $cm^{-1}$

FIG. 2

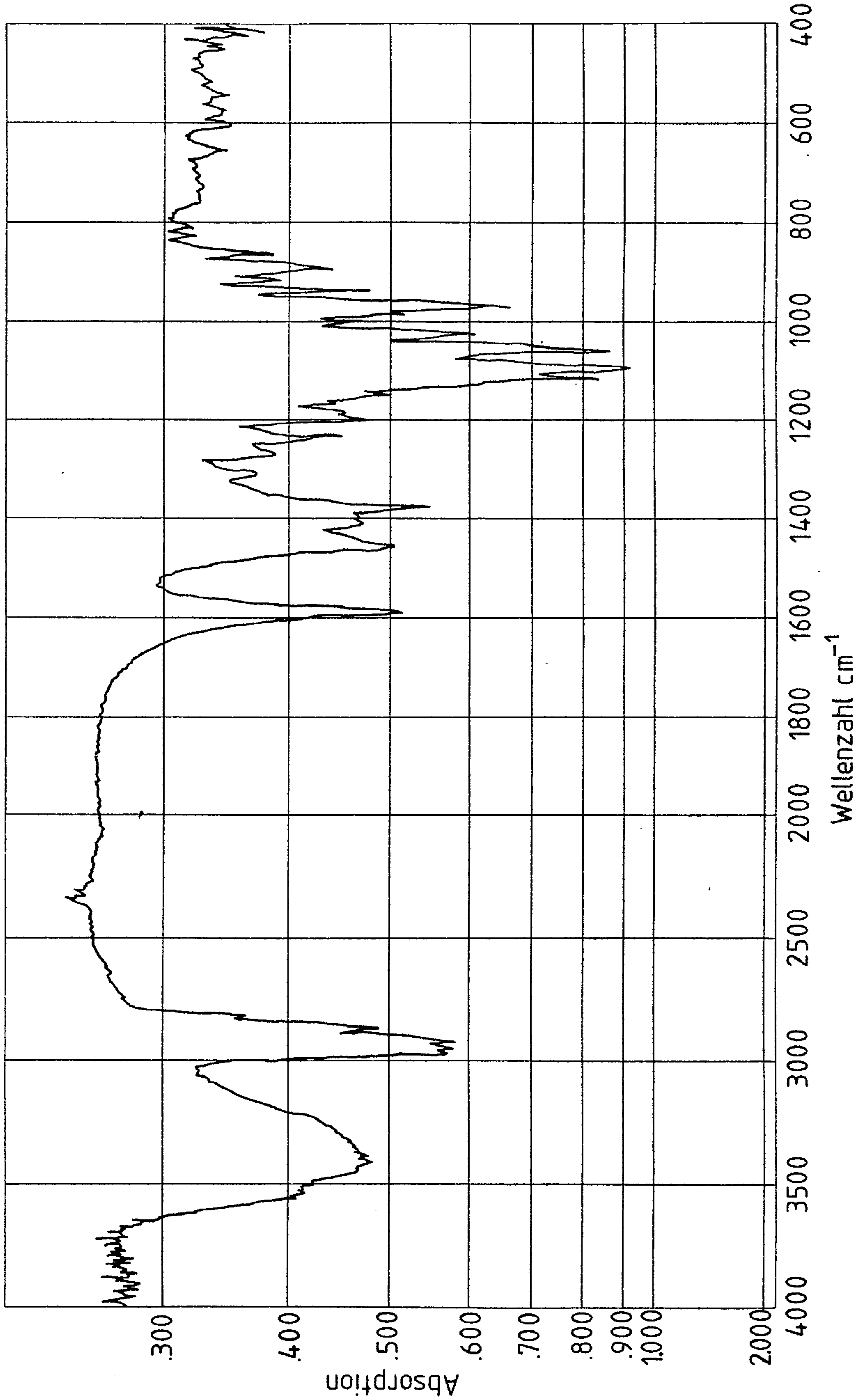
Absorption
.300
.400
.500
.600
.700
.800
.900
1.000
2.000
4000
3500
3000
2500
2000
1800
1600
1400
1200
1000
800
600
400
Wellenzahl $cm^{-1}$

FIG. 3

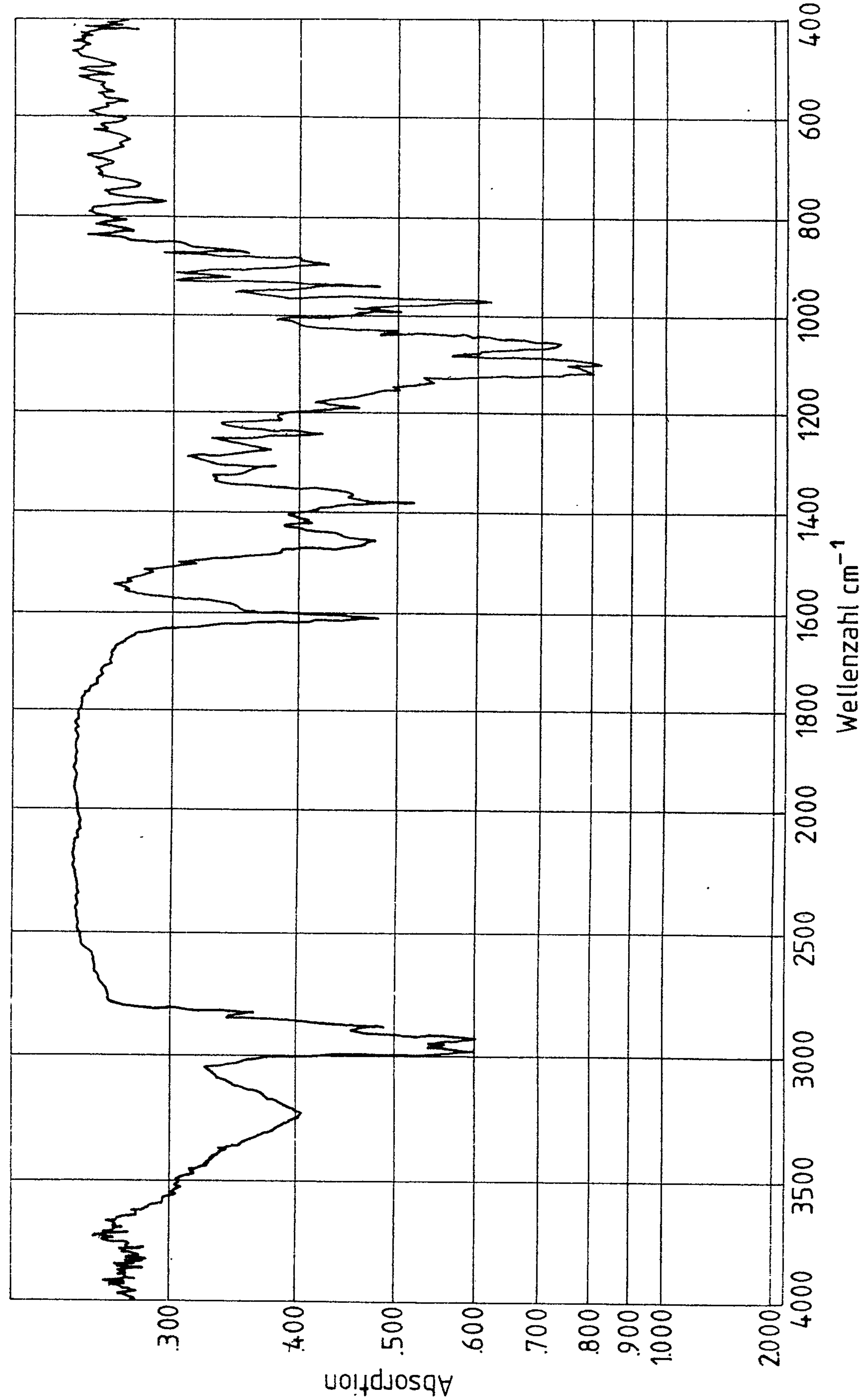
4000
3500
3000
2500
2000
1800
1600
1400
1200
1000
800
600
400
Wellenzahl $cm^{-1}$
3.00
4.00
5.00
6.00
7.00
8.00
9.00
1.000
2.000
Absorption

FIG. 4